(19) Europäisches Patentamt
European Patent Office
Office européen des brevets

(11) Numéro de publication : **0 073 704**
**B1**

(12) **FASCICULE DE BREVET EUROPÉEN**

(45) Date de publication du fascicule du brevet : **12.11.86**

(21) Numéro de dépôt : **82401501.0**

(22) Date de dépôt : **06.08.82**

(51) Int. Cl.[4] : **C 07 D409/04**, C 07 D405/04, C 07 D411/04, A 61 K 31/33

# (54) Nouveaux nitriles hétérocycliques, leur préparation et leur utilisation pour la préparation de médicaments.

(30) Priorité : **11.08.81 FR 8115527**

(43) Date de publication de la demande :
**09.03.83 Bulletin 83/10**

(45) Mention de la délivrance du brevet :
**12.11.86 Bulletin 86/46**

(84) Etats contractants désignés :
**AT BE CH DE FR GB IT LI LU NL SE**

(56) Documents cités :
**FR-A- 1 458 902**
**FR-A- 2 100 970**
**FR-A- 2 258 178**
**FR-A- 2 452 488**

(73) Titulaire : **RHONE-POULENC SANTE**
**Les Miroirs 18 Avenue d'Alsace**
**F-92400 Courbevoie Cedex (FR)**

(72) Inventeur : **Aloup, Jean-Claude**
**53, rue Marcel Risser**
**F-94290 Villeneuve-le-Roi (FR)**
Inventeur : **Bouchaudon, Jean**
**23, avenue des Saules**
**F-91390 Morsang-sur-Orge (FR)**
Inventeur : **Farge, Daniel**
**30, rue des Pins Sylvestres**
**F-94320 Thiais (FR)**
Inventeur : **James, Claude**
**31 bis, avenue Gambetta**
**F-75020 Paris (FR)**

(74) Mandataire : **Le Goff, Yves et al**
**RHONE-POULENC RECHERCHES Brevets Pharma**
**25, Quai Paul Doumer**
**F-92408 Courbevoie (FR)**

EP 0 073 704 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne de nouveaux nitriles hétérocycliques utiles pour la préparation de thioformamides douées de propriétés thérapeutiques.

On connait déjà par le brevet FR-A-2 252 488 thioformamides ayant des propriétés anti-ulcères mais le brevet n'enseigne pas qu'elles peuvent être obtenues à partir d'un nitrile ; de plus, ces thioformamides sont différentes de celles que l'on peut obtenir à partir des nitriles selon l'invention. On connaît également par les brevets FR-A-2 100 970 et 2 258 178 des nitriles hétérocycliques mais ils ne peuvent pas conduire aux thioformamides que l'on peut obtenir à partir des nitriles selon la présente invention.

La présente invention concerne donc de nouveaux nitriles permettant de préparer des thioformamides non connues dans l'art antérieur.

Plus particulièrement la présente invention concerne de nouveaux nitriles hétérocycliques de formule générale :

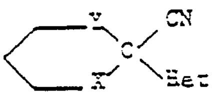

(I)

Dans la formule générale (I) :

— soit Het représente le radical quinolyle-2, X représente un atome de soufre et Y représente une liaison de valence,

— soit Het représente le radical pyridyle-2, X représente un atome de soufre et Y représente un atome de soufre ou un radical méthylène,

— soit Het représente le radical pyridyle-2, X représente un atome d'oxygène et Y représente une liaison de valence,

— soit Het représente un radical hétérocyclique à caractère aromatique contenant un ou deux atomes d'azote choisi parmi pyridyle-3, pyridyle-4, pyridazinyle-3, pyrazinyle ou quinolyle-3, et ou bien X représente un atome de soufre ou d'oxygène et Y représente une liaison de valence ou un radical méthylène, ou bien X et Y représentent un atome de soufre.

Selon l'invention, les produits de formule générale (I) dans laquelle :

— soit Het représente un radical hétérocyclique à caractère aromatique contenant un ou deux atomes d'azote choisi parmi pyridyle-3, pyridyle-4, pyridazinyle-3, pyrazinyle, quinolyle-3, X représente un atome de soufre et Y représente un atome de soufre, une liaison de valence ou un radical méthylène.

— soit Het représente le radical pyridyle-2, X représente un atome de soufre et Y représente un atome de soufre ou un radical méthylène peuvent être obtenus par action d'un dérivé de formule générale :

$$Z—(CH_2)_n\ SCN \qquad (II)$$

dans laquelle Z représente un atome d'halogène (tel que le chlore, le brome ou l'iode) ou un reste d'ester réactif (tel que mésyloxy ou tosyloxy) et n représente un nombre entier égal à 3 ou 4, ou bien Z représente un radical thiocyanato et n est égal à 3, sur un nitrile de formule générale :

$$Het\ CH_2\ CN \qquad (III)$$

dans laquelle Het a la définition correspondante.

On opère dans un solvant organique tel qu'un hydrocarbure aromatique (benzène, toluène, xylène) ou un solvant chloré (chlorure de méthylène) en présence d'un réactif de transfert de phase tel qu'un hydroxyde ou un sel de tétraalcoylammonium ou de trialcoylbenzylammonium dans une solution aqueuse d'un hydroxyde alcalin, à une température comprise entre 20 et 70 °C, sous vive agitation. Dans la pratique, on utilise de préférence le chlorure de triéthylbenzylammonium dans une solution aqueuse de soude à 50 %.

Les thiocyanates de formule générale (II) peuvent être préparés selon les méthodes de R. ADAMS et J.B. CAMPBELL, J. Am. Chem. Soc., *72*, 128 (1950) ou de L. HAGELBERT, Chem. Ber. *23*, 1083 (1890).

Les produits de formule générale (III) peuvent être préparés selon la méthode décrite par S. OKUDA et M.M. ROBISON, J. Am. Chem. Soc. *81*, 740 (1959).

Selon l'invention, les produits de formule générale (I) dans laquelle :

— soit Het représente un radical hétérocyclique à caractère aromatique contenant un ou deux atomes d'azote choisi parmi pyridyle-3, pyridyle-4, pyridazinyle-3, pyrazinyle ou quinolyle, X représente un atome de soufre ou d'oxygène et Y représente une liaison de valence ou un radical méthylène.

— soit Het représente le radical pyridyle-2, X représente un atome de soufre et Y représente un radical méthylène, ou X représente un atome d'oxygène et Y représente une liaison de valence peuvent être obtenus par cyclisation d'un dérivé de formule générale :

$$Het-\underset{\displaystyle CN}{\underset{|}{CH}}-(CH_2)_m-X-H \qquad (IV)$$

dans laquelle X et Het ont les définitions correspondantes et m est un nombre entier égal à 3 ou 4.

On opère généralement dans un solvant organique tel qu'un hydrocarbure aromatique (benzène, toluène, xylène) ou un solvant chloré (chlorure de méthylène) en présence de tétrachlorure de carbone et d'un réactif de transfert de phase tel qu'un hydroxyde ou un sel de tétraalcoylammonium ou de trialcoylbenzylammonium dans une solution aqueuse d'un hydroxyde alcalin, à une température comprise entre 20 et 70 °C, sous vive agitation. Dans la pratique, on utilise de préférence le chlorure de triéthylbenzylammonium dans une solution aqueuse de soude à 50 %.

Les dérivés de formule générale (IV) peuvent être préparés par condensation d'un dérivé de formule générale :

$$Z_1(CH_2)_m—X—H \qquad (V)$$

dans laquelle $Z_1$ représente un atome d'halogène (tel que chlore, brome ou iode) ou un reste d'ester réactif (tel que mésyloxy ou tosyloxy) et X et m ont les définitions correspondantes, sur un nitrile de formule générale (III).

Il est entendu que la fonction thiol ou alcool des produits de formule générale (V) doit être préalablement bloquée par un radical protecteur labile puis libérée après condensation sur le nitrile de formule générale (III).

Comme radical protecteur, on peut utiliser tout radical stable en milieu alcalin et pouvant être facilement éliminé sans toucher au reste de la molécule. Il est particulièrement avantageux d'effectuer le blocage par un radical tétrahydropyrannyle [selon L.A. PAQUETTE et M.K. SCOTT, J. Am. Chem. Soc. *94*, 6751 (1972)].

La condensation du produit de formule générale (V) préalablement bloqué sur le nitrile de formule générale (III) peut être effectuée par toute méthode connue en soi pour condenser un dérivé halogéné ou un ester réactif (mésylate ou tosylate) sur un dérivé à méthylène actif. Il est particulièrement avantageux d'opérer dans un solvant organique tel qu'un hydrocarbure aromatique (benzène, toluène, xylène) ou un solvant chloré (chlorure de méthylène) en présence d'un réactif de transfert de phase tel qu'un hydroxyde ou un sel de tétraalcoylammonium ou de trialcoylbenzylammonium dans une solution aqueuse d'un hydroxyde alcalin, à une température comprise entre 20 et 70 °C, sous vive agitation. Dans la pratique, on utilise de préférence le chlorure de triéthylbenzylammonium dans une solution de soude aqueuse à 50 %.

Les nouveaux produits de formule générale (I) sont utiles notamment comme intermédiaires pour la préparation de thioformamides de formule générale :

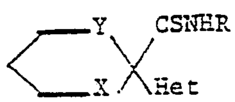

(IX)

dans laquelle R représente un atome d'hydrogène ou un radical alcoyle contenant 1 à 4 atomes de carbone en chaı←ne droite ou ramifiée et Het, X et Y sont définis comme précédemment.

Pour obtenir les produits de formule générale (IX), les produits selon l'invention peuvent être mis en œuvre de la façon suivante :

— On hydrolyse le nitrile de formule générale (I) en acide correspondant de formule générale :

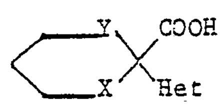

(X)

par toute méthode connue en soi pour transformer un nitrile en acide sans toucher au reste de la molécule.

— On condense l'ammoniac ou une amine de formule générale :

$$R'—NH_2 \qquad (XI)$$

dans laquelle R' représente un radical alcoyle contenant 1 à 4 atomes de carbone en chaîne droite ou ramifiée sur l'acide de formule générale (X) dans laquelle les symboles sont définis comme précédemment, pour obtenir une formamide de formule générale :

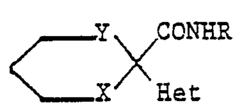

(XII)

Dans la pratique, on fait généralement réagir l'ammoniac ou l'amine de formule générale (XI) avec

l'acide de formule générale (X) en présence de N,N'-carbonyldiimidazole ou de dicyclohexylcarbodiimide à une température voisine de 20 °C dans un solvant organique inerte tel que l'acétonitrile, le chlorure de méthylène, le diméthylformamide ou l'acétate d'éthyle, ou avec un halogénure de l'acide de formule générale (X) à une température comprise entre 0 et 50 °C, au sein d'un solvant tel que l'éther, le tétrahydrofuranne ou un solvant chloré, en présence d'un accepteur d'acide qui peut être un excès d'ammoniac ou d'amine de formule générale (XI), ou bien la pyridine ou la triéthylamine.

— On transforme la formamide de formule générale (XII) en thioformamide de formule générale (IX) au moyen d'un réactif de thionation.

Comme réactif de thionation, il est particulièrement avantageux d'utiliser $P_2S_5$ ou mieux le réactif de PEDERSEN-LAWESSON [bis(méthoxy-4 phényl)-2,4 dithioxo-2,4 dithia-1,3 diphospha-2,4 étanne] préparé selon B. S. PEDERSEN, S. SCHEIBYE, N. H. NILSSON et S. O. LAWESSON, Bull. Soc. Chim. Belge, *87*, 223 (1978). On opère généralement dans un solvant organique inerte vis-à-vis du réactif de thionation, tel qu'un hydrocarbure aromatique (benzène, toluène, xylène), un éther (dioxanne), un solvant chloré (chlorobenzène) ou la pyridine, à une température comprise entre 50 °C et la température de reflux du mélange réactionnel.

Les nouveaux produits selon la présente invention ainsi que les produits de formule générale (IX) préparés à partir de ceux-ci peuvent être purifiés par les méthodes physiques habituelles, notamment la cristallisation et la chromatographie.

Les produits de formule générale (IX) préparés à partir des produits selon l'invention présentent des propriétés pharmacologiques particulièrement intéressantes associées à une faible toxicité. Ils manifestent une activité anti-ulcère, anti-sécrétoire et/ou une activité régulatrice du système cardiovasculaire (anti-hypertenseurs).

Présentent plus particulièrement des propriétés anti-ulcères de type antisécrétoire les produits de formule générale (IX) dans laquelle :

— soit Het représente le radical quinolyle-2, X représente un atome de soufre et Y représente une liaison de valence,

— soit Het représente le radical pyridyle-2, X représente un atome de soufre et Y représente un atome de soufre ou un radical méthylène,

— soit Het représente le radical pyridyle-2, X représente un atome d'oxygène et Y représente une liaison de valence.

Ces propriétés peuvent être mises en évidence chez le rat, à des doses comprises entre 1 et 100 mg/kg par voie orale, en particulier dans la technique de ROSSI et coll. C. R. Soc. Biol., *150*, 2124 (1956) et celle de SHAY et coll., Gastroenterology, *5*, 43 (1945). Leur dose létale ($DL_{50}$) chez la souris, est généralement supérieure à 300 mg/kg par voie orale.

Sont plus spécialement intéressants comme anti-ulcères les produits de formule générale (IX) dans laquelle le symbole Het tel qu'il vient d'être défini, représente le radical pyridyle-2, quinolyle-2 ou pyridazinyle-3.

Sont d'un intérêt tout particulier :

— le N-méthyl (quinolyl-2)-2 tétrahydrothiophènecarbothioamide-2
— le N-méthyl (pyridyl-2)-2 tétrahydrothiopyrannecarbothioamide-2
— le N-méthyl (pyridyl-2)-2 dithianne-1,3 carbothioamide-2
— le N-méthyl (pyridyl-2)-2 tétrahydrofurannecarbothioamide-2
— le (pyridyl-2)-2 dithianne-1,3 carbothioamide-2
— le N-méthyl (pyridazinyl-3)-2 tétrahydrothiophènecarbothioamide-2.

Présentent plus particulièrement des propriétés régulatrices du système cardiovasculaire (anti-hypertenseurs) les produits de formule générale (IX) dans laquelle Het représente un radical hétérocyclique à caractère aromatique contenant un ou deux atomes d'azote, rattaché par un atome de carbone situé en β d'un atome d'azote et choisi parmi pyridyle-3, pyridyle-4, pyridazinyle-3, pyrazinyle ou quinolyle-3, et ou bien X représente un atome de soufre ou d'oxygène et Y représente une liaison de valence ou un radical méthylène, ou bien X et Y représentent un atome de soufre. A des doses comprises entre 0,1 et 100 mg par voie orale, ils abaissent la pression artérielle chez le rat spontanément hypertendu (rat SHR) de souche OKAMOTO-AOKI. L'utilisation du rat spontanément hypertendu pour l'étude des produits anti-hypertenseurs est décrite par J. L. ROBA, Lab. Anim. Sci. *26*, 305 (1976).

Sont plus spécialement intéressants comme anti-hypertenseurs, les produits de formule générale (IX) dans laquelle le symbole Het représente le radical pyridyle-3 ou quinolyle-3.

D'un intérêt tout particulier comme anti-hypertenseurs sont :

— le N-méthyl (pyridyl-3)-2 tétrahydrothiophènecarbothioamide-2
— le N-méthyl (pyridyl-3)-2 tétrahydrothiopyrannecarbothioamide-2
— le N-méthyl (quinolyl-3)-2 tétrahydrothiophènecarbothioamide-2.

Les exemples suivants, donnés à titre non limitatif, montrent comment l'invention peut être mise en pratique.

Exemple 1

A une solution de 156 g de pyridyl-3 acétonitrile dans 430 $cm^3$ de toluène, on ajoute sous agitation

une solution de 216 g de soude en pastilles dans 216 $cm^3$ d'eau distillée puis 6,5 g de chlorure de triéthylbenzylammonium. On ajoute ensuite goutte à goutte en 20 minutes, en maintenant la température en dessous de 52 °C, une solution de 282 g de thiocyanate de bromo-3 propyle dans 430 $cm^3$ de toluène. Après 1 heure d'agitation à 67 °C et refroidissement à 30 °C, le mélange réactionnel est décanté et la phase aqueuse est diluée avec 200 $cm^3$ d'eau distillée et extraite 2 fois avec 600 $cm^3$ au total d'acétate d'éthyle. Les phases organiques sont réunies et lavées 2 fois avec 600 $cm^3$ au total d'eau distillée puis 2 fois avec 500 $cm^3$ au total d'une solution aqueuse d'acide chlorhydrique 5N. Les extraits chlorhydriques sont réunis, lavés 2 fois avec 600 $cm^3$ au total d'acétate d'éthyle et neutralisés par addition de 500 $cm^3$ d'une solution d'ammoniaque 10N en présence de 500 $cm^3$ d'acétate d'éthyle. Après décantation, la phase aqueuse est extraite 2 fois avec 500 $cm^3$ au total d'acétate d'éthyle. Les extraits organiques sont réunis, lavés 3 fois avec 600 $cm^3$ au total d'eau distillée, séchés sur du sulfate de sodium anhydre et concentrés à sec sous pression réduite (20 mm de mercure ; 2,7 kPa) à 60 °C. Le produit obtenu (215 g) est dissous dans 1 600 $cm^3$ d'oxyde d'isopropyle bouillant et la solution, additionnée de 2 g de noir décolorant, est filtrée à chaud puis conservée pendant 3 heures à 0 °C. Les cristaux apparus sont séparés par filtration, lavés avec 150 $cm^3$ d'oxyde d'isopropyle et séchés sous pression réduite (20 mm de mercure ; 2,7 kPa) à une température voisine de 20 °C. On obtient ainsi 145 g de (pyridyl-3)-2 tétrahydrothiophènecarbonitrile-2 fondant à 45 °C.

Le thiocyanate de bromo-3 propyle peut être préparé selon la méthode décrite dans la littérature par R. ADAMS et J. B. CAMPBELL [J. Am. Chem. Soc., *72*, 128 (1950)].

Le pyridyl-3 acétonitrile peut être préparé selon la méhode décrite dans la littérature par S. OKUDA et M. M. ROBISON ([J. Am. Chem. Soc., *81*, 740 (1959)].

## Exemple 2

Une solution de 27,5 g d'α-(hydroxy-3 propyl) pyridyl-3 acétonitrile dans 30 $cm^3$ de toluène est ajoutée, en 10 minutes et à une température ne dépassant pas 40 °C, à un mélange agité de 120 $cm^3$ de tétrachlorure de carbone, 100 g de soude en pastilles préalablement dissous dans 100 $cm^3$ d'eau distillée et 2 g de chlorure de triéthylbenzylammonium. Après 1 heure d'agitation à une température voisine de 20 °C, on ajoute 250 $cm^3$ d'eau distillée et extrait le mélange réactionnel 3 fois avec 600 $cm^3$ au total de chlorure de méthylène. Les extraits organiques sont réunis, lavés 2 fois avec 400 $cm^3$ au total d'eau distillée, séchés sur du sulfate de sodium anhydre et concentrés à sec sous pression réduite (20 mm de mercure ; 2,7 kPa) à 40 °C. Le produit obtenu (13 g) est chromatographié sur 75 g de gel de silice neutre contenus dans une colonne de 3,4 cm de diamètre. On élue avec un mélange cyclohexane-acétate d'éthyle (70-30 en volumes) en recueillant une fraction de 200 $cm^3$ et une fraction de 3,5 litres. Cette dernière est concentrée à sec sous pression réduite (20 mm de mercure ; 2,7 kPa) à 40 °C. On obtient ainsi 10 g de (pyridyl-3)-2 tétrahydrofurannecarbonitrile-2 sous la forme d'une huile jaune.

(Rf = 0,6 ; chromatographie sur couche mince de gel de silice ; solvant : acétate d'éthyle).

L'α-(hydroxy-3 propyl) pyridyl-3 acétonitrile peut être préparé de la façon suivante :

Une solution de 43 g d'α-[(tétrahydropyrannyl-2 oxy)-3 propyl] pyridyl-3 acétonitrile dans 800 $cm^3$ d'une solution aqueuse d'acide chlorhydrique 1N est conservée pendant environ 16 heures à une température voisine de 20 °C. On extrait ensuite le mélange réactionnel 3 fois avec 750 $cm^3$ au total d'acétate d'éthyle puis on ajoute lentement, en maintenant la température en dessous de 20 °C, une solution aqueuse de soude 10N de façon à ajuster le pH du milieu entre 8 et 9. On extrait 3 fois avec 900 $cm^3$ au total d'acétate d'éthyle et les extraits organiques sont réunis, lavés avec 250 $cm^3$ d'eau distillée, séchés sur du sulfate de sodium anhydre et concentrés à sec sous pression réduite (20 mm de mercure ; 2,7 kPa) à 40 °C. On obtient ainsi 27,5 g d'α-(hydroxy-3 propyl) pyridyl-3-acétonitrile sous la forme d'une huile jaune.

(Rf = 0,35 ; chromatographie sur couche mince de gel de silice ; solvant : acétate d'éthyle).

L'α-[(tétrahydropyrannyl-2 oxy)-3 propyl] pyridyl-3 acétonitrile peut être préparé de la façon suivante :

A une solution de 23,9 g de pyridyl-3 acétonitrile dans 50 $cm^3$ de toluène, on ajoute 40 g de soude en pastilles en solution dans 40 $cm^3$ d'eau distillée puis 2,5 g de chlorure de triéthylbenzylammonium. Le mélange est agité et on ajoute goutte à goutte, en 15 minutes, en maintenant la température en dessous de 50 °C, une solution de 51,3 g de bromo-1 (tétrahydropyrannyl-2 oxy)-3 propane dans 30 $cm^3$ de toluène. Après 1 heure 30 minutes d'agitation à une température voisine de 40 °C puis 15 heures à une température voisine de 20 °C, on ajoute 200 $cm^3$ d'eau distillée et extrait le mélange 3 fois avec 450 $cm^3$ au total de chlorure de méthylène. Les extraits organiques sont réunis, lavés 3 fois avec 450 $cm^3$ au total d'eau distillée, séchés sur du sulfate de sodium anhydre et concentrés à sec sous pression réduite (20 mm de mercure ; 2,7 kPa) à 40 °C. Le produit obtenu (56,7 g) est chromatographié sur 500 g de gel de silice neutre contenus dans une colonne de 5 cm de diamètre. On élue successivement avec 3 litres d'un mélange cyclohexane-acétate d'éthyle (80-20 en volumes) et avec 9 litres d'un mélange cyclohexane-acétate d'éthyle (30-70 en volumes) en recueillant 1 fraction de 3 litres et 1 fraction de 9 litres. Cette dernière est concentrée à sec sous pression réduite (20 mm de mercure ; 2,7 kPa) à 50 °C. On obtient ainsi 43 g d'α-[(tétrahydropyrannyl-2 oxy)-3 propyl] pyridyl-3 acétonitrile sous la forme d'une huile jaune.

(Rf = 0,55 ; chromatographie sur couche mince de gel de silice ; solvant : acétate d'éthyle).

Le bromo-1 (tétrahydropyrannyl-2 oxy)-3 propane peut être préparé comme décrit dans la littérature par L. A. PAQUETTE et M. K. SCOTT [J. Am. Chem. Soc., *94*, 6751 (1972)].

Exemple 3

A une solution de 40 g de pyridyl-3 acétonitrile dans 120 $cm^3$ de toluène on ajoute sous agitation, en maintenant la température en dessous de 30 °C, une solution de 65,3 g de soude en pastilles dans 65 $cm^3$ d'eau distillée puis 1,63 g de chlorure de triéthylbenzylammonium. On ajoute ensuite goutte à goutte, en 25 minutes, en maintenant la température en dessous de 45 °C, une solution de 73,5 g de thiocyanate de bromo-4 butyle dans 120 $cm^3$ de toluène. Après 1 heure 30 minutes d'agitation à 54 °C et refroidissement à 25 °C, le mélange réactionnel est décanté. La phase organique est lavée 5 fois avec 750 $cm^3$ au total d'eau distillée puis avec 250 $cm^3$ d'une solution aqueuse d'acide chlorhydrique 2N. Les extraits chlorhydriques sont réunis, lavés 3 fois avec 450 $cm^3$ au total d'éther éthylique, additionnés de 0,7 g de noir décolorant, filtrés et alcalinisés par addition de 60 $cm^3$ d'une solution aqueuse de soude 10N. La phase aqueuse est extraite 4 fois avec 800 $cm^3$ au total d'éther éthylique. Les extraits éthérés sont réunis, lavés 3 fois avec 450 $cm^3$ au total d'eau distillée, séchés sur du sulfate de magnésium anhydre, additionnés de 1 g de noir décolorant, filtrés et concentrés à sec sous pression réduite (20 mm de mercure ; 2,7 kPa) à 50 °C. Le produit obtenu (35 g) est chromatographié sur une colonne de 3,6 cm de diamètre contenant 180 g de silice (0,063-0,2 mm) en éluant d'abord par 3 litres de chlorure de méthylène pur, puis par 500 $cm^3$ d'un mélange chlorure de méthylène/méthanol (99,5-0,5 en volumes) et en recueillant des fractions de 500 $cm^3$. La première fraction est éliminée. Les 6 fractions suivantes sont réunies et concentrées à sec sous pression réduite (20 mm de mercure ; 2,7 kPa) à 40 °C. On obtient ainsi 18,3 g de (pyridyl-3)-2 tétrahydrothiopyrannecarbonitrile-2 sous la forme d'une huile orangée qui cristallise dans l'oxyde d'isopropyle (F. = 75 °C).

[Rf = 0,47 ; chromatographie sur couche mince de gel de silice ; solvant : cyclohexane-acétate d'éthyle (50-50 en volumes)].

Le thiocyanate de bromo-4 butyle peut être préparé selon la méthode décrite dans la littérature par R. ADAMS et D. C. BLOMSTROM [J. Am. Chem. Soc. *75*, 2375 (1953)].

Le pyridyl-3 acétonitrile peut être préparé selon la méthode décrite dans la littérature par S. OKUDA et M. M. ROBISON [J. Am. Chem. Soc. *81*, 740 (1959)].

Les produits de formule générale (I) peuvent être mis en œuvre pour la préparation des produits de formule générale (IX) en opérant comme décrit dans les exemples d'utilisation suivants.

Exemple d'utilisation 1

Une solution de 34 g de (pyridyl-3)-2 tétrahydrothiophènecarbonitrile-2 dans 320 $cm^3$ d'une solution aqueuse d'acide chlorhydrique 10N est chauffée pendant 3 heures à 70 °C sous agitation. Après 15 heures d'agitation supplémentaire à une température voisine de 20 °C, le mélange réactionnel est concentré à sec sous pression réduite (1 mm de mercure ; 0,13 kPa) à 70 °C. Le produit obtenu (48 g) est dissous dans 150 $cm^3$ d'eau distillée et la solution est additionnée de 20 $cm^3$ d'une solution d'ammoniaque 10N. Le précipité apparu est séparé par filtration, lavé avec 25 cm3 d'eau distillée puis 2 fois avec 100 $cm^3$ au total d'oxyde d'isopropyle et séché sous pression réduite (20 mm de mercure ; 2,7 kPa) à une température voisine de 20 °C. On obtient ainsi 31 g de produit que l'on dissout dans 210 $cm^3$ d'éthanol bouillant ; la solution, additionnée de 0,5 g de noir décolorant, est filtrée à chaud puis conservée pendant 1 heure à 0 °C. Les cristaux apparus sont séparés par filtration, lavés avec 20 $cm^3$ d'éthanol puis 2 fois avec 40 $cm^3$ au total d'oxyde d'isopropyle et séchés sous pression réduite (20 mm de mercure ; 2,7 kPa) à une température voisine de 20 °C. On redissout le produit obtenu (18 g) dans 87 $cm^3$ d'une solution aqueuse de soude 1N. La solution est lavée 3 fois avec 150 $cm^3$ au total de chloroforme, additionnée de 0,5 g de noir décolorant et filtrée. On ajoute alors au filtrat environ 6 $cm^3$ d'une solution aqueuse d'acide chlorhydrique 10N de façon à ajuster le pH à 4. Les cristaux qui apparaissent sont séparés par filtration, lavés avec 10 $cm^3$ d'eau distillée et séchés sous pression réduite (1 mm de mercure ; 0,13 kPa) à une température voisine de 20 °C. Le produit obtenu (13,9 g) est dissous dans 130 $cm^3$ d'éthanol bouillant et la solution est filtrée à chaud puis conservée pendant 3 heures à une température voisine de 5 °C. Les cristaux apparus sont séparés par filtration, lavés 2 fois avec 40 $cm^3$ au total d'oxyde d'isopropyle et séchés sous pression réduite (1 mm de mercure ; 0,13 kPa) à 60 °C. On obtient finalement 11,5 g d'acide (pyridyl-3)-2 tétrahydrothiophènecarboxylique-2 fondant à 184 °C.

58,5 g d'acide (pyridyl-3)-2 tétrahydrothiophènecarboxylique-2 obtenus comme précédemment sont ajoutés par petites fractions en 15 minutes à 90 $cm^3$ de chlorure de thionyle additionnés de 0,1 $cm^3$ de diméthylformamide. Le mélange réactionnel est ensuite agité à l'ébullition pendant 2 heures, refroidi à une température voisine de 20 °C puis concentré à sec sous pression réduite (20 mm de mercure ; 2,7 kPa) à 70 °C. On obtient ainsi 75 g de chlorhydrate de chloroformyl-2 (pyridyl-3)-2 tétrahydrothiophène brut sous la forme d'un solide jaune employé aussitôt sans autre purification.

3,5 g de chlorhydrate de chloroformyl-2 (pyridyl-3)-2 tétrahydrothiophène obtenus ainsi sont dissous dans 35 $cm^3$ de chlorure de méthylène. On sature alors la solution obtenue (2 heures) par un courant de monométhylamine anhydre en maintenant la température du mélange réactionnel à environ 20 °C. Après

16 heures d'agitation à la même température, on ajoute 40 $cm^3$ d'une solution aqueuse saturée de bicarbonate de sodium, décante et extrait la phase aqueuse 3 fois avec 75 $cm^3$ au total de chlorure de méthylène. Les extraits organiques sont réunis, lavés 3 fois avec 60 $cm^3$ au total d'eau distillée, séchés sur du sulfate de sodium anhydre et concentrés à sec sous pression réduite (20 mm de mercure ; 2,7 kPa) à 50 °C. Le produit obtenu (1,9 g) est dissous dans 175 $cm^3$ d'oxyde d'isopropyle bouillant et la solution additionnée de 0,3 g de noir décolorant est filtrée à chaud puis conservée après refroidissement pendant 3 heures à une température voisine de 5 °C. Les cristaux apparus sont séparés par filtration, lavés avec 2 $cm^3$ d'oxyde d'isopropyle et séchés sous pression réduite (20 mm de mercure ; 2,7 kPa) à 20 °C. Le produit obtenu (1,2 g) auquel on a ajouté 1,2 g de produit préparé de la même manière dans une autre opération est dissous dans 250 $cm^3$ d'oxyde d'isopropyle bouillant et la solution, additionnée de 0,2 g de noir décolorant, est filtrée à chaud puis conservée après refroidissement pendant 15 heures à une température voisine de 5 °C. Les cristaux apparus sont séparés par filtration, lavés 2 fois avec 6 $cm^3$ au total d'oxyde d'isopropyle et séchés sous pression réduite (1 mm de mercure ; 0,13 kPa) à 60 °C. On obtient ainsi 1,95 g de N-méthyl (pyridyl-3)-2 tétrahydrothiophènecarboxamide-2 fondant à 123 °C.

A une solution de 6,8 g de N-méthyl (pyridyl-3)-2 tétrahydrothiophènecarboxamide-2 ainsi préparé dans 90 $cm^3$ de toluène, on ajoute 9,3 g de bis (méthoxy-4 phényl)-2,4 dithioxo-2,4 dithia-1,3 diphospha-2,4 étanne. La suspension obtenue est chauffée à l'ébullition pendant 3 heures. Le mélange est ensuite refroidi à une température voisine de 20 °C, additionnée de 100 $cm^3$ d'eau distillée, de 120 $cm^3$ d'acétate d'éthyle et de 10 $cm^3$ d'une solution aqueuse d'ammoniaque 10N. Après décantation, la phase aqueuse est extraite 2 fois avec 240 $cm^3$ au total d'acétate d'éthyle. Les extraits organiques sont réunis et lavés 3 fois avec 150 $cm^3$ au total d'eau distillée, séchés sur du sulfate de sodium anhydre et concentrés sous pression réduite (20 mm de mercure ; 2,7 kPa) à 60 °C. Le produit obtenu (7,5 g) est dissous dans 40 $cm^3$ d'éthanol bouillant et la solution, additionnée de 0,1 g de noir décolorant, est filtrée à chaud puis conservée, après refroidissement, pendant 1 heure à une température voisine de 5 °C. Les cristaux apparus sont séparés par filtration, lavés avec 4 $cm^3$ d'éthanol puis 2 fois avec 20 $cm^3$ au total d'oxyde d'isopropyle et séchés sous pression réduite (1 mm de mercure ; 0,13 kPa) à 60 °C. On obtient ainsi 4,9 g de N-méthyl (pyridyl-3)-2 tétrahydrothiophènecarbothioamide-2 fondant à 133 °C.

### Exemple d'utilisation 2

Une solution de 10 g de (pyridyl-3)-2 tétrahydrofurannecarbonitrile-2 dans 100 $cm^3$ d'une solution aqueuse d'acide chlorhydrique 10N est chauffée pendant 3 heures à une température comprise entre 60 °C et 70 °C puis concentrée à sec sous pression réduite (20 mm de mercure ; 2,7 kPa) à 60 °C.

Le mélange de chlorhydrate de l'acide intermédiaire et de chlorure d'ammonium ainsi obtenu est dissous dans 50 $cm^3$ de chlorure de thionyle contenant 0,1 $cm^3$ de diméthylformamide. Après 1 heure 15 minutes de chauffage à l'ébullition, on concentre le mélange réactionnel à sec sous pression réduite (20 mm de mercure ; 2,7 kPa) à 40 °C puis on ajoute 200 $cm^3$ de chlorure de méthylène. Les cristaux insolubles sont éliminés par filtration et le filtrat est concentré à sec sous pression réduite (20 mm de mercure ; 2,7 kPa) à 40 °C. On obtient ainsi 12,9 g de chlorhydrate de chloroformyl-2 (pyridyl-3)-2 tétrahydrofuranne sous la forme d'une huile brun-clair.

Ce chlorhydrate est dissous dans 130 $cm^3$ de chlorure de méthylène. Dans la solution obtenue, on fait barboter jusqu'à saturation (3 heures) un courant de monométhylamine anhydre en maintenant la température à environ 20 °C. On ajoute ensuite 100 $cm^3$ d'eau distillée et, après décantation, la phase organique est lavée avec 100 $cm^3$ d'eau distillée, séchée sur du sulfate de sodium anhydre et concentrée à sec sous pression réduite (20 mm de mercure ; 2,7 kPa) à 40 °C. Le produit obtenu (6,8 g) est chromatographié sur 70 g de gel de silice neutre contenus dans une colonne de 3 cm de diamètre. On élue avec 3 litres d'un mélange cyclohexane-acétate d'éthyle (70-30 en volumes) puis avec 5 litres d'acétate d'éthyle en recueillant une fraction de 3 litres et une de 5 litres. Cette dernière est concentrée à sec sous pression réduite (20 mm de mercure ; 2,7 kPa) à 50 °C. On obtient ainsi 5,2 g de N-méthyl (pyridyl-3)-2 tétrahydrofurannecarboxamide-2 brut fondant à 107 °C.

A une solution de 5 g de N-méthyl (pyridyl-3)-2 tétrahydrofurannecarboxamide-2 préparé comme indiqué ci-dessus dans 120 $cm^3$ de toluène, on ajoute 7,7 g de bis(méthoxy-4 phényl)-2,4 dithioxo-2,4 dithia-1,3 diphospha-2,4 étanne et la suspension obtenue est chauffée à l'ébullition pendant 2 heures. Le mélange réactionnel est ensuite refroidi à une température voisine de 20 °C puis additionné de 100 $cm^3$ d'eau distillée, 100 $cm^3$ d'une solution aqueuse d'ammoniaque 10N et 100 $cm^3$ d'acétate d'éthyle. Après décantation, la phase aqueuse est extraite avec 50 $cm^3$ d'acétate d'éthyle et les extraits organiques sont réunis, lavés avec 100 $cm^3$ d'eau distillée, séchés sur du sulfate de sodium anhydre et concentrés à sec sous pression réduite (20 mm de mercure ; 2,7 kPa) à 50 °C. Le produit obtenu (6,7 g) est chromatographié sur 75 g de gel de silice neutre contenus dans une colonne de 3 cm de diamètre. On élue successivement avec 1 500 $cm^3$ d'un mélange cyclohexane-acétate d'éthyle (80-20 en volumes) et avec 2 400 $cm^3$ d'un mélange cyclohexane-acétate d'éthyle (60-40 en volumes) en recueillant 13 fractions de 300 $cm^3$. Les fractions 6 à 13 sont réunies et concentrées à sec sous pression réduite (20 mm de mercure ; 2,7 kPa) à 50 °C. Le produit obtenu (3,5 g) est dissous dans 50 $cm^3$ de chlorure de méthylène et la solution est extraite 5 fois avec 125 $cm^3$ au total d'une solution aqueuse d'acide chlorhydrique 2N. Les extraits acides sont réunis, lavés 2 fois avec 40 $cm^3$ de chlorure de méthylène et amenés à un pH voisin de

8 par addition de 25 $cm^3$ d'une solution aqueuse de soude 1ON. Le mélange est ensuite extrait 4 fois avec 200 $cm^3$ au total de chlorure de méthylène et les extraits organiques sont réunis, lavés 2 fois avec 100 $cm^3$ au total d'eau distillée, séchés sur du sulfate de sodium anhydre et concentrés à sec sous pression réduite (20 mm de mercure ; 2,7 kPa) à 40 °C. Le produit obtenu (2 g) est dissous dans 40 $cm^3$ d'un mélange bouillant d'oxyde d'isopropyle-éthanol (80-20 en volumes) et la solution, additionnée de 0,1 g de noir décolorant, est filtrée à chaud puis conservée après refroidissement pendant 1 heure à 0 °C. Les cristaux apparus sont séparés par filtration, lavés 2 fois avec 10 $cm^3$ au total d'oxyde d'isopropyle et séchés sous pression réduite (1 mm de mercure ; 0,13 kPa) à 60 °C. On obtient ainsi 1,5 g de N-méthyl (pyridyl-3)-2 tétrahydrofurannecarbothioamide-2 fondant à 128 °C.

## Exemple d'utilisation 3

Une solution de 18 g de (pyridyl-3)-2 tétrahydrothiopyrannecarbonitrile-2 dans 180 $cm^3$ d'une solution aqueuse d'acide chlorhydrique 12N est chauffée sous agitation pendant 20 heures à 80 °C. Après refroidissement à une température voisine de 20 °C, le mélange réactionnel est concentré à sec sous pression réduite (20 mm de mercure ; 2,7 kPa) à 70 °C. Le produit brut obtenu est dissous dans 50 $cm^3$ d'eau distillée et la solution ainsi préparée est additionnée à 0,1 g de noir décolorant, filtrée, additionnée d'une solution d'ammoniaque 7,5N (environ 17,5 $cm^3$) jusqu'à obtention d'un pH de 3,7 et conservée 1 heure à une température voisine de 5 °C. Le précipité apparu est séparé par filtration, lavé 3 fois avec 30 $cm^3$ au total d'eau distillée glacée et séché sous pression réduite (20 mm de mercure ; 2,7 kPa) à une température voisine de 20 °C en présence de potasse en pastilles puis à l'air. On obtient ainsi 16,6 g d'acide (pyridyl-3)-2 tétrahydrothiopyrannecarboxylique-2 fondant à 193-194 °C.

16,4 g d'acide (pyridyl-3)-2 tétrahydrothiopyrannecarboxylique-2 ainsi obtenus sont ajoutés par petites fractions en 30 minutes à 25 $cm^3$ de chlorure de thionyle additionnés de 0,05 $cm^3$ de diméthylformamide. Le mélange réactionnel est ensuite agité à l'ébullition pendant 2 heures et 30 minutes, refroidi à une température voisine de 20 °C puis concentré à sec sous pression réduite (20 mm de mercure ; 2,7 kPa) à 70 °C. On obtient ainsi 26 g de chlorhydrate de chloroformyl-2 (pyridyl-3)-2 tétrahydrothiopyranne brut sous la forme d'une huile orange employée aussitôt sans autre purification.

Ce produit est dissous dans 70 $cm^3$ de chlorure de méthylène. On sature alors la solution obtenue (2 heures) par un courant de monométhylamine anhydre en maintenant la température du mélange réactionnel à environ 20 °C. Après 16 heures d'agitation à la même température, on sépare le précipité obtenu par filtration et le lave 3 fois avec 90 $cm^3$ au total de chlorure de méthylène. Les filtrats organiques sont réunis, lavés 5 fois avec 250 $cm^3$ au total d'eau distillée, séchés sur sulfate de magnésium anhydre, additionnés de 0,2 g de noir décolorant, filtrés et concentrés à sec sous pression réduite (20 mm de mercure ; 2,7 kPa) à 60 °C. On obtient ainsi un résidu huileux (16,3 g) qui est chromatographié sur une colonne de 3,4 cm de diamètre contenant 165 g de silice (0,063-0,2 mm) en éluant par de l'acétate d'éthyle et en recueillant des fractions de 100 $cm^3$. Les 4 premières fractions sont éliminées. Les 10 fractions suivantes sont réunies et concentrées à sec sous pression réduite (20 mm de mercure ; 2,7 kPa) à 60 °C. On obtient ainsi 14 g de produit brut que l'on dissout dans 20 $cm^3$ d'acétonitrile bouillant. Cette solution est conservée après refroidissement pendant 1 heure à une température voisine de 5 °C. Les cristaux apparus sont séparés par filtration, lavés 3 fois avec 20 $cm^3$ au total d'oxyde d'isopropyle refroidi à 5 °C environ et séchés sous pression réduite (20 mm de mercure ; 2,7 kPa) à une température voisine de 20 °C en présence de potasse en pastilles. On obtient ainsi 11,3 g de N-méthyl (pyridyl-3)-2 tétrahydrothiopyrannecarboxamide-2 fondant à 109 °C.

On ajoute 7,5 g de bis(méthoxy-4 phényl)-2,4 dithioxo-2,4 dithia-1,3 diphospha-2,4 étanne à une suspension de 5,9 g de N-méthyl (pyridyl-3)-2 tétrahydrothiopyrannecarboxamide-2 ainsi préparés dans 75 $cm^3$ de toluène. La suspension obtenue est chauffée à l'ébullition sous agitation pendant 6 heures. Après 16 heures supplémentaires d'agitation à une température voisine de 20 °C, le mélange réactionnel est additionné de 80 $cm^3$ d'eau distillée, de 80 $cm^3$ d'acétate d'éthyle et de 8 $cm^3$ d'une solution d'ammoniaque 15N. Après décantation, la phase aqueuse est extraite 3 fois avec 300 $cm^3$ au total d'acétate d'éthyle. Les extraits organiques sont réunis et lavés 4 fois avec 320 $cm^3$ au total d'eau distillée, séchés sur du sulfate de magnésium anhydre, additionnés de 0,5 g de noir décolorant, filtrés et concentrés à sec sous pression réduite (20 mm de mercure ; 2,7 kPa) à 70 °C. Le produit obtenu (6,4 g) est dissous dans 15 $cm^3$ d'éthanol bouillant. Cette solution est conservée, après refroidissement, pendant 30 minutes à une température voisine de 5 °C. Les cristaux apparus sont séparés par filtration, lavés 2 fois avec 14 $cm^3$ au total d'éthanol refroidi à une température voisine de 5 °C puis 2 fois avec 20 $cm^3$ au total d'oxyde d'isopropyle et séchés sous pression réduite (20 mm de mercure ; 2,7 kPa) à une température voisine de 20 °C en présence de potasse en pastilles. On obtient ainsi 4,6 g de produit brut fondant à 120 °C.

Ce produit est dissous dans 40 $cm^3$ d'une solution aqueuse d'acide chlorhydrique 0,5 N. La solution ainsi obtenue est extraite 3 fois avec 75 $cm^3$ au total d'acétate d'éthyle, additionnée de 0,1 g de noir décolorant, filtrée et alcalinisée par 12 $cm^3$ d'une solution aqueuse de soude 2N en présence de 25 $cm^3$ d'acétate d'éthyle. Après décantation, la phase aqueuse est extraite 2 fois avec 50 $cm^3$ au total d'acétate d'éthyle. Les extraits organiques sont réunis, lavés 3 fois avec 60 $cm^3$ au total d'eau distillée, séchés sur sulfate de magnésium anhydre, filtrés et concentrés à sec sous pression réduite (20 mm de mercure ;

2,7 kPa) à 60 °C. Le produit obtenu (3,3 g) est dissous dans 9 $cm^3$ d'éthanol bouillant. Cette solution est conservée, après refroidissement, pendant 30 minutes à une température voisine de 5 °C. Les cristaux apparus sont séparés par filtration, lavés 2 fois avec 6 $cm^3$ au total d'éthanol refroidi à une température voisine de 50 °C et séchés sous pression réduite (1 mm de mercure ; 0,13 kPa) à 60 °C. On obtient ainsi 2,6 g de N-méthyl (pyridyl-3)-2 tétrahydrothiopyrannecarbothioamide-2 fondant à 124 °C.

En opérant comme à l'exemple d'utilisation 1 mais à partir des nitriles correspondants, on peut préparer les produits suivants :

Exemple d'utilisation 4

N-méthyl (pyridyl-4)-2 tétrahydrothiophènecarbothioamide-2 fondant à 178 °C.

Exemple d'utilisation 5

N-méthyl (quinolyl-2)-2 tétrahydrothiophènecarbothioamide-2 fondant à 124 °C.

Exemple d'utilisation 6

N-méthyl (pyridyl-2)-2 tétrahydrothiopyrannecarbothioamide-2 fondant à 153 °C.

Exemple d'utilisation 7

(Pyridyl-2)-2 dithianne-1,3 carbothioamide-2 fondant à 214 °C.

Exemple d'utilisation 8

N-méthyl (pyridyl-2)-2 dithianne-1,3 carbothioamide-2 fondant à 159 °C.

Exemple d'utilisation 9

N-méthyl (pyrazinyl-2)-2 tétrahydrothiophènecarbothioamide-2 fondant à 127 °C.

Exemple d'utilisation 10

N-méthyl (pyridyl-2)-2 tétrahydrofurannecarbothioamide-2 fondant à 115 °C.

Exemple d'utilisation 11

N-méthyl (pyridazinyl-3)-2 tétrahydrothiophènecarbothioamide-2 fondant à 199 °C.

Exemple d'utilisation 12

N-méthyl (quinolyl-3)-2 tétrahydrothiophènecarbothioamide-2 fondant à 159 °C.

**Revendications** (Pour les Etats contractants : BE, CH, DE, FR, GB, IT, LI, LU, NL, SE)

1. Un nitrile hétérocyclique caractérisé en ce qu'il répond à la formule générale :

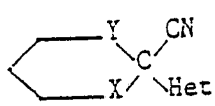

dans laquelle

— soit Het représente le radical quinolyle-2, X représente un atome de soufre et Y représente une liaison de valence,

— soit Het représente le radical pyridyle-2, X représente un atome de soufre et Y représente un atome de soufre ou un radical méthylène,

— soit Het représente le radical pyridyle-2, X représente un atome d'oxygène et Y représente une liaison de valence,

— soit Het représente un radical hétérocyclique à caractère aromatique contenant un ou deux atomes d'azote choisi parmi pyridyle-3, pyridyle-4, pyridazinyle-3, pyrazinyle ou quinolyle-3, et ou bien X représente un atome de soufre ou d'oxygène et Y représente une liaison de valence ou un radical méthylène, ou bien X et Y représentent un atome de soufre.

2. Un procédé de préparation d'un nitrile hétérocyclique selon la revendication 1 dans la formule duquel

— soit Het représente un radical hétérocyclique à caractère aromatique contenant un ou deux atomes d'azote choisi parmi pyridyle-3, pyridyle-4, pyridazinyle-3, pyrazinyle, quinolyle-3, X représente un atome de soufre et Y représente un atome de soufre, une liaison de valence ou un radical méthylène

— soit Het représente le radical pyridyle-2, X représente un atome de soufre et Y représente un atome de soufre ou un radical méthylène, caractérisé en ce que l'on fait réagir un dérivé de formule générale :

$$Z—(CH_2)_nSCN$$

dans laquelle Z représente un atome d'halogène ou un reste d'ester réactif et n représente un nombre entier égal à 3 ou 4, ou bien Z représente un radical thiocyanato et n est égal à 3, sur un nitrile de formule générale :

$$Het—CH_2CN$$

dans laquelle Het a la définition correspondante, puis on isole le produit obtenu.

3. Un procédé de préparation d'un nitrile hétérocyclique selon la revendication 1 dans la formule duquel :

— soit Het représente un radical hétérocyclique à caractère aromatique contenant un ou deux atomes d'azote choisi parmi pyridyle-3, pyridyle-4, pyridazinyle-3, pyrazinyle ou quinolyle, X représente un atome de soufre ou d'oxygène et Y représente une liaison de valence ou un radical méthylène

— soit Het représente le radical pyridyle-2, X représente un atome de soufre et Y représente un radical méthylène, ou X représente un atome d'oxygène et Y représente une liaison de valence, caractérisé en ce que l'on cyclise un dérivé de formule générale :

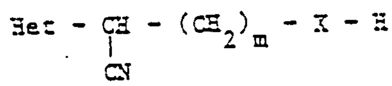

dans laquelle Het et X ont les définitions correspondantes et m est un nombre entier égal à 3 ou 4, en présence de tétrachlorure de carbone et d'un réactif de transfert de phase, puis on isole le produit obtenu.

4. Utilisation d'un nitrile hétérocyclique selon la revendication 1 pour la préparation d'un dérivé de la thioformamide de formule :

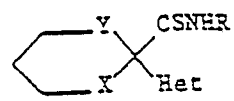

dans laquelle Het, X et Y sont définis comme dans la revendication 1 et R représente un atome d'hydrogène ou un radical alcoyle contenant 1 à 4 atomes de carbone en chaîne droite ou ramifiée, caractérisée en ce que l'on hydrolyse le nitrile selon la revendication 1 en acide correspondant, on condense l'ammoniac ou une amine de formule générale

$$R'—NH_2$$

dans laquelle R' représente un radical alcoyle contenant 1 à 4 atomes de carbone en chaîne droite ou ramifiée pour obtenir une formamide de formule générale :

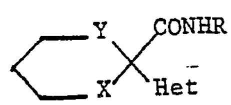

dans laquelle les symboles ont les définitions correspondantes puis on fait réagir sur celle-ci un réactif de thionation, et on isole le produit obtenu.

**Revendications** (pour l'Etat contractant AT)

1. Procédé de préparation d'un nouveau nitrile hétérocyclique de formule générale :

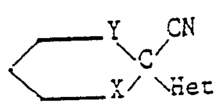

dans laquelle

— soit Het représente le radical quinolyle-2, X représente un atome de soufre et Y représente une liaison de valence,

— soit Het représente le radical pyridyle-2, X représente un atome de soufre et Y représente un atome de soufre ou un radical méthylène,

— soit Het représente le radical pyridyle-2, X représente un atome d'oxygène et Y représente une liaison de valence,

— soit Het représente un radical hétérocyclique à caractère aromatique contenant un ou deux atomes d'azote choisi parmi pyridyle-3, pyridyle-4, pyridazinyle-3, pyrazinyle ou quinolyle-3, et ou bien X représente un atome de soufre ou d'oxygène et Y représente une liaison de valence ou un radical méthylène, ou bien X et Y représentent un atome de soufre, caractérisé en ce que, pour la préparation d'un nitrile hétérocyclique de formule précédente dans laquelle :

— soit Het représente un radical hétérocyclique à caractère aromatique contenant un ou deux atomes d'azote choisi parmi pyridyle-3, pyridyle-4, pyridazinyle-3, pyrazinyle, quinolyle-3, X représente un atome de soufre et Y représente un atome de soufre, une liaison de valence ou un radical méthylène

— soit Het représente le radical pyridyle-2, X représente un atome de soufre et Y représente un atome de soufre ou un radical méthylène, on fait réagir un dérivé de formule générale :

$$Z—(CH_2)_nSCN$$

dans laquelle Z représente un atome d'halogène ou un reste d'ester réactif et n représente un nombre entier égal à 3 ou 4, ou bien Z représente un radical thiocyanato et n est égal à 3, sur un nitrile de formule générale :

$$Het—CH_2CN$$

dans laquelle Het a la définition correspondante ou bien pour la préparation d'un nitrile hétérocyclique de formule précédente dans laquelle :

— soit Het représente un radical hétérocyclique à caractère aromatique contenant un ou deux atomes d'azote choisi parmi pyridyle-3, pyridyle-4, pyridazinyle-3, pyrazinyle ou quinolyle, X représente un atome de soufre ou d'oxygène et Y représente une liaison de valence ou un radical méthylène

— soit Het représente le radical pyridyle-2, X représente un atome de soufre et Y représente un radical méthylène, ou X représente un atome d'oxygène et Y représente une liaison de valence, on cyclise un dérivé de formule générale :

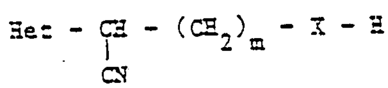

dans laquelle Het et X ont les définitions correspondantes et m est un nombre entier égal à 3 ou 4, en présence de tétrachlorure de carbone et d'un réactif de transfert de phase.

2. Utilisation d'un nitrile hétérocyclique préparé selon la revendication 1 pour la préparation d'un dérivé de la thioformamide de formule :

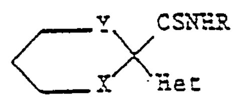

dans laquelle Het, X et Y sont définis comme dans la revendication 1 et R représente un atome d'hydrogène ou un radical alcoyle contenant 1 à 4 atomes de carbone en chaîne droite ou ramifiée, caractérisée en ce que l'on hydrolise le nitrile selon la revendication 1 en acide correspondant, on condense l'ammoniac ou une amine de formule générale

$$R'—NH_2$$

dans laquelle R' représente un radical alcoyle contenant 1 à 4 atomes de carbone en chaîne droite ou ramifiée pour obtenir une formamide de formule générale :

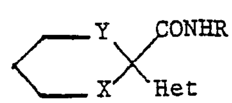

dans laquelle les symboles ont les définitions correspondantes puis on fait réagir sur celle-ci un réactif de thionation, et on isole le produit obtenu.

**Claims** (for the Contracting States : BE, CH, DE, FR, GB, IT, LI, LU, NL, SE)

1. A heterocyclic nitrile, characterized in that it corresponds to the general formula :

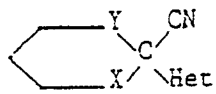

in which
— either Het denotes a 2-quinolyl radical, X denotes a sulphur atom and Y denotes a valency bond,
— or Het denotes a 2-pyridyl radical, X denotes a sulphur atom and Y denotes a sulphur atom or a methylene radical,
— or Het denotes a 2-pyridyl radical, X denotes an oxygen atom and Y denotes a valency bond,
— or Het denotes a heterocyclic radical of aromatic nature containing one or two nitrogen atoms, chosen from 3-pyridyl, 4-pyridyl, 3-pyridazinyl, pyrazinyl or 3-quinolyl, and either X denotes a sulphur or oxygen atom and Y denotes a valency bond or a methylene radical, or alternatively X and Y denote a sulphur atom.

2. A process for preparing a heterocyclic nitrile according to Claim 1, in the formula of which
— either Het denotes a heterocyclic radical of aromatic nature containing one or two nitrogen atoms, chosen from 3-pyridyl, 4-pyridyl, 3-pyridazinyl, pyrazinyl or 3-quinolyl, X denotes a sulphur atom and Y denotes a sulphur atom, a valency bond or a methylene radical,
— or Het denotes a 2-pyridyl radical, X denotes a sulphur atom and Y denotes a sulphur atom or a methylene radical, characterized in that a derivative of general formula :

$$Z-(CH_2)_nSCN$$

in which Z denotes a halogen atom or a reactive ester residue and n denotes an integer equal to 3 or 4, or alternatively Z denotes a thiocyanato radical and n equals 3, is reacted with a nitrile of general formula :

$$Het-CH_2CN$$

in which Het has the corresponding definition, and the product obtained is then isolated.

3. A process for preparing a heterocyclic nitrile according to Claim 1, in the formula of which :
— either Het denotes a heterocyclic radical of aromatic nature containing one or two nitrogen atoms, chosen from 3-pyridyl, 4-pyridyl, 3-pyridazinyl, pyrazinyl or quinolyl, X denotes a sulphur or oxygen atom and Y denotes a valency bond or a methylene radical,
— or Het denotes a 2-pyridyl radical, X denotes a sulphur atom and Y denotes a methylene radical, or X denotes an oxygen atom and Y denotes a valency bond, characterized in that a derivative of general formula :

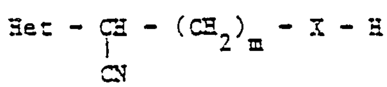

in which Het and X have the corresponding definitions and m is an integer equal to 3 or 4, is cyclized in the presence of carbon tetrachloride and a phase transfer reagent, and the product obtained is then isolated.

4. Use of a heterocyclic nitrile according to Claim 1 for the preparation of a thioformamide derivative of formula :

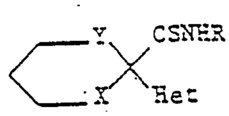

in which Het, X and Y are defined as in Claim 1 and R denotes a hydrogen atom or a straight-chain or branched alkyl radical containing 1 to 4 carbon atoms, characterized in that the nitrile according to Claim 1 is hydrolysed to the corresponding acid, ammonia or an amine of general formula

$$R'-NH_2$$

in which R′ denotes a straight-chain or branched alkyl radical containing 1 to 4 carbon atoms, is condensed therewith to obtain a formamide of general formula :

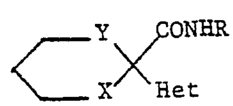

in which the symbols have the corresponding definitions, the latter compound is then reacted with a thionation reagent and the product obtained is isolated.

**Claims** (for the Contracting State AT)

1. Process for preparing a new heterocyclic nitrile of general formula :

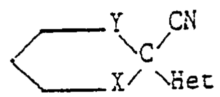

in which

— either Het denotes a 2-quinolyl radical, X denotes a sulphur atom and Y denotes a valency bond,

— or Het denotes a 2-pyridyl radical, X denotes a sulphur atom and Y denotes a sulphur atom or a methylene radical,

— or Het denotes a 2-pyridyl radical, X denotes an oxygen atom and Y denotes a valency bond,

— or Het denotes a heterocyclic radical of aromatic nature containing one or two nitrogen atoms, chosen from 3-pyridyl, 4-pyridyl, 3-pyridazinyl, pyrazinyl or 3-quinolyl, and either X denotes a sulphur or oxygen atom and Y denotes a valency bond or a methylene radical, or alternatively X and Y denote a sulphur atom, characterized in that, for the preparation of a heterocyclic nitrile of the above formula in which :

— either Het denotes a heterocyclic radical of aromatic nature containing one or two nitrogen atoms, chosen from 3-pyridyl, 4-pyridyl, 3-pyridazinyl, pyrazinyl or 3-quinolyl, X denotes a sulphur atom and Y denotes a sulphur atom, a valency bond or a methylene radical,

— or Het denotes a 2-pyridyl radical, X denotes a sulphur atom and Y denotes a sulphur atom or a methylene radical, a derivative of general formula :

$$Z—(CH_2)_nSCN$$

in which Z denotes a halogen atom or a reactive ester residue and n denotes an integer equal to 3 or 4, or alternatively Z denotes a thiocyanato radical and n equals 3, is reacted with a nitrile of general formula :

$$Het—CH_2CN$$

in which Het has the corresponding definition, or for the preparation of a heterocyclic nitrile of the above formula in which :

— either Het denotes a heterocyclic radical of aromatic nature containing one or two nitrogen atoms, chosen from 3-pyridyl, 4-pyridyl, 3-pyridazinyl, pyrazinyl or quinolyl, X denotes a sulphur or oxygen atom and Y denotes a valency bond or a methylene radical,

— or Het denotes a 2-pyridyl radical, X denotes a sulphur atom and Y denotes a methylene radical, or X denotes an oxygen atom and Y denotes a valency bond, a derivative of general formula :

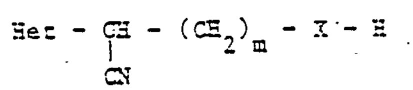

in which Het and X have the corresponding definitions and m is an integer equal to 3 or 4, is cyclized in the presence of carbon tetrachloride and a phase transfer reagent.

2. Use of a heterocyclic nitrile prepared according to Claim 1 for the preparation of a thioformamide derivative of formula :

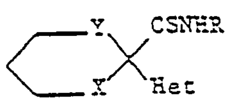

in which Het, X and Y are defined as in Claim 1 and R denotes a hydrogen atom or a straight-chain or branched alkyl radical containing 1 to 4 carbon atoms, characterized in that the nitrile according to Claim 1 is hydrolysed to the corresponding acid, ammonia or an amine of general formula

$$R'—NH_2$$

in which R' denotes a straight-chain or branched alkyl radical containing 1 to 4 carbon atoms, is condensed therewith to obtain a formamide of general formula :

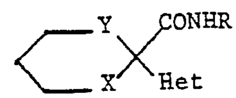

in which the symbols have the corresponding definitions, the latter compound is then reacted with a thionation reagent and the product obtained is isolated.

**Patentansprüche** (für die Vertragsstaaten : BE, CH, DE, FR, GB, IT, LI, LU, NL, SE)

1. Heterocyclisches Nitril, dadurch gekennzeichnet, daß es der allgemeinen Formel

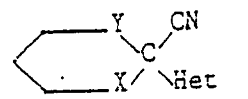

entspricht, worin

— entweder Het den Chinolyl-2-Rest bedeutet, X ein Schwefelatom, und Y eine Valenzbindung bedeutet,

— oder Het den Pyridyl-2-Rest bedeutet, X ein Schwefelatom und Y ein Schwefelatom oder einen Methylenrest bedeutet,

— oder Het den Pyridyl-2-Rest, X ein Sauerstoffatom und Y eine Valenzbindung bedeutet,

— oder Het einen heterocyclischen Rest mit aromatischem Charakter, der ein oder zwei Stickstoffatome enthält, ausgewählt aus Pyridyl-3, Pyridyl-4, Pyridazinyl-3, Pyrazinyl oder Chinolyl-3, bedeutet, und entweder X ein Schwefel- oder Sauerstoffatom und Y eine Valenzbindung oder einen Methylenrest bedeutet, oder X und Y bedeuten ein Schwefelatom.

2. Verfahren zur Herstellung eines heterocyclischen Nitrils gemäß Anspruch 1, in dessen Formel

— entweder Het einen heterocyclischen Rest mit aromatischem Charakter, enthaltend ein oder zwei Stickstoffatome ausgewählt unter Pyridyl-3, Pyridyl-4, Pyridazinyl-3, Pyrazinyl, Chinolyl-3, bedeutet, X ein Schwefelatom und Y ein Schwefelatom, eine Valenzbindung oder einen Methylenrest bedeutet,

— oder Het den Pyridyl-2-Rest bedeutet, X ein Schwefelatom und Y ein Schwefelatom oder einen Methylenrest bedeutet, dadurch gekennzeichnet, daß man ein Derivat der allgemeinen Formel

$$Z—(CH_2)_nSCN$$

worin Z ein Halogenatom oder einen Rest eines reaktionsfähigen Esters bedeutet, und n eine ganze Zahl gleich 3 oder 4 bedeutet, oder Z einen Thiocyanatorest bedeutet und n gleich 3 ist, auf ein Nitril der allgemeinen Formel

$$Het—CH_2CN$$

worin Het die entsprechende Definition hat, einwirken läßt, und das erhaltene Produkt dann isoliert.

3. Verfahren zur Herstellung eines heterocyclischen Nitrils gemäß Anspruch 1, in dessen Formel

— entweder Het einen heterocyclischen Rest mit aromatischem Charakter, enthaltend ein oder zwei Stickstoffatome ausgewählt unter Pyridyl-3, Pyridyl-4, Pyridazinyl-3, Pyrazinyl oder Chinolyl, bedeutet, X ein Schwefel- oder Sauerstoffatom und Y eine Valenzbindung oder einen Methylenrest bedeutet,

— oder Het den Pyridyl-2-Rest bedeutet, X ein Schwefelatom darstellt und Y einen Methylenrest bedeutet, oder X ein Sauerstoffatom und Y eine Valenzbindung bedeutet, dadurch gekennzeichnet, daß man ein Derivat der allgemeinen Formel

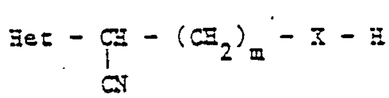

worin Het und X die entsprechenden Bedeutungen haben und m eine ganze Zahl gleich 3 oder 4 ist, in Gegenwart von Tetrachlorkohlenstoff und einem Phasentransferreaktionsmittel cyclisiert und das erhaltene Produkt dann isoliert:

4. Verwendung eines heterocyclischen Nitrils gemäß Anspruch 1 zur Herstellung eines Derivats des Thioformamids der Formel

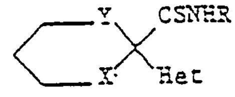

worin Het, X und Y wie in Anspruch 1 definiert sind und R ein Wasserstoffatom oder einen Alkylrest mit 1

bis 4 Kohlenstoffatomen in gerader oder verzweigter Kette bedeutet, dadurch gekennzeichnet, daß man das Nitril gemäß Anspruch 1 zur entsprechenden Säure hydrolysiert, daß man Ammoniak oder ein Amin der allgemeinen Formel

$$R'—NH_2$$

worin R' einen Alkylrest mit 1 bis 4 Kohlenstoffatomen in gerader oder verzweigter Kette bedeutet, zur Erzielung eines Formamids der allgemeinen Formel

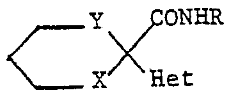

worin die Symbole die entsprechenden Bedeutungen haben, kondensiert und dann auf dieses ein Thionierungsmittel einwirken läßt, und das erhaltene Produkt isoliert.

**Patentansprüche** (für den Vertragsstaat AT)

1. Verfahren zur Herstellung eines neuen heterocyclischen Nitrils der allgemeinen Formel :

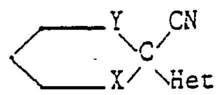

in welcher

— Het entweder den 2-Chinolylrest darstellt, X ein Schwefelatom darstellt und Y eine Valenzbindung darstellt

— oder Het den 2-Pyridylrest darstellt, X ein Schwefelatom darstellt und Y ein Schwefelatom oder einen Methylenrest darstellt,

— oder Het den 2-Pyridylrest darstellt, X ein Sauerstoffatom darstellt und Y eine Valenzbindung darstellt,

— oder Het einen heterocyclischen Rest aromatischen Charakters mit ein oder zwei Stickstoffatomen, ausgewählt aus 3-Pyridyl, 4-Pyridyl, 3-Pyridazinyl, Pyrazinyl oder 3-Chinolyl, darstellt und X entweder ein Schwefel- oder Sauerstoffatom darstellt und Y eine Valenzbindung oder einen Methylenrest darstellt oder X und Y ein Schwefelatom darstellen, dadurch gekennzeichnet, daß man zur Herstellung eines heterocyclischen Nitrils der obigen Formel, in welcher

— Het entweder einen heterocyclischen Rest aromatischen Charakters mit einem oder zwei Stickstoffatomen, ausgewählt aus 3-Pyridyl, 4-Pyridyl-, 3-Pyridazinyl-, Pyrazinyl-, 3-Chinolyl-, darstellt, X ein Schwefelatom darstellt und Y ein Schwefelatom, eine Valenzbindung oder einen Methylenrest darstellt,

— oder Het den 2-Pyridylrest darstellt, X ein Schwefelatom darstellt und Y ein Schwefelatom oder einen Methylenrest darstellt, ein Derivat der allgemeinen Formel :

$$Z—(CH_2)_nSCN,$$

in welcher Z ein Halogenatom oder einen reaktiven Esterrest darstellt und n eine ganze Zahl gleich 3 oder 4 darstellt, oder Z einen Thiocyanatrest darstellt und n gleich 3 ist, mit einem Nitril der allgemeinen Formel :

$$Het—CH_2CN,$$

in welcher Het die entsprechende Bedeutung hat, umsetzt oder zur Herstellung eines heterocyclischen Nitrils der obigen Formel, in welcher :

— Het entweder einen heterocyclischen Rest aromatischen Charakters mit ein oder zwei Stickstoffatomen, ausgewählt aus 3-Pyridyl, 4-Pyridyl-, 3-Pyridazinyl-, Pyrazinyl oder Chinolyl, darstellt X ein Schwefel- oder Sauerstoffatom darstellt und Y eine Valenzbindung oder einen Methylenrest darstellt,

— oder Het den 2-Pyridylrest darstellt, X ein Schwefelatom darstellt und Y einen Methylenrest darstellt oder X ein Sauerstoffatom darstellt und Y eine Valenzbindung darstellt, ein Derivat der allgemeinen Formel :

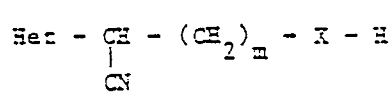

in welcher Het und X die entsprechenden Bedeutungen haben und m eine ganze Zahl gleich 3 oder 4 ist, in Gegenwart von Tetrachlorkohlenstoff und eines Phasenübertragungsmittels cyclisiert.

2. Verwendung eines heterocyclischen Nitrils, hergestellt nach Anspruch 1 zur Herstellung eines Thioformamidderivats der Formel :

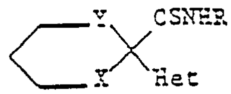

in welcher Het, X und Y die im Anspruch 1 angegebene Bedeutung haben und R ein Wasserstoffatom oder einen Alkylrest darstellt, der 1 bis 4 Kohlenstoffatome in gerader oder verzweigter Kette enthält, dadurch gekennzeichnet, daß man das Nitril nach Anspruch 1 zur entsprechenden Säure hydrolysiert, daß man Ammoniak oder ein Amin der allgemeinen Formel :

$$R'—NH_2$$

in welcher R' einen geradkettigen oder verzweigten Alkylrest mit 1 bis 4 Kohlenstoffatomen darstellt, unter Bildung eines Formamids der allgemeinen Formel :

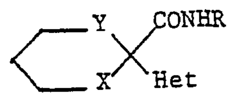

in welcher die Symbole die entsprechenden Bedeutungen haben, kondensiert, daß man dann auf dieses ein Thionierungsmittel einwirken läßt und das erhaltene Produkt isoliert.